(19) Europäisches Patentamt
European Patent Office
Office européen des brevets

(11) Publication number: **0 040 536 B1**

(12) **EUROPEAN PATENT SPECIFICATION**

(45) Date of publication of patent specification: 08.08.84

(51) Int. Cl.$^3$: C 12 P 7/66 //C12R1/645

(21) Application number: 81302196.1

(22) Date of filing: 18.05.81

(54) **Method for producing coenzyme Q10.**

(30) Priority: 16.05.80 JP 65739/80

(43) Date of publication of application:
25.11.81 Bulletin 81/47

(45) Publication of the grant of the patent:
08.08.84 Bulletin 84/32

(84) Designated Contracting States:
CH DE FR GB IT LI NL SE

(56) References cited:
FR - A - 2 342 337
FR - A - 2 369 342
GB - A - 1 343 066

CHEMICAL ABSTRACTS, vol. 93, no. 13, September 29, 1980, page 512, abstract no. 130575x COLUMBUS, Ohio (US)

CHEMICAL ABSTRACTS, vol. 77, no. 11, September 11, 1972, page 358, abstract no. 73688e COLUMBUS, Ohio (US)

CHEMICAL ABSTRACTS, vol. 70, no. 19, May 12, 1969, page 87, abstract no. 85145u COLUMBUS, Ohio (US)

(73) Proprietor: NAGOYA UNIVERSITY
1 Furo-Cho
Chikusa-ku Nagoya City (JP)

(72) Inventor: Kaneko, Yasuyuki
8-299 Ueda-Sanshichikawahara Tenpaku-Cho
Tenpaku-ku Nagoya City (JP)
Inventor: Ito, Masao
10-7 Imaike-Cho 3-Chome
Anjyo City (JP)

(74) Representative: Woods, Geoffrey Corlett et al,
J.A. KEMP & CO. 14 South Square Gray's Inn
London WC1R 5EU (GB)

(56) References cited:
CHEMICAL ABSTRACTS, vol. 92, no. 7, February 18, 1980, page 335, abstract no. 54801e COLUMBUS, Ohio (US)

CHEMICAL ABSTRACTS, vol. 91, no. 6, August 6, 1979, page 297 abstract no. 44174t COLUMBUS, Ohio (US)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 040 536 B1

## Description

The present invention relates to a method for producing coenzyme $Q_{10}$.

Recently, coenzyme $Q_{10}$ has been interested as a raw material for medicines for curing various diseases such as heart diseases, hypertension, tumor etc. and can be produced by various microorganisms. For example, Journal of Fermentation Technology, *47,* p 551 (1969) discloses a method of improving productivity of coenzyme Q by adding as a component of culture medium p-hydroxybenzoic acid (hereinafter abbreviated to POBA) which is a precursor in a biosynthetic pathway of coenzyme Q. However, the method has a defect that the produced coenzyme Q is $Q_9$ and not $Q_{10}$. Coenzyme $Q_9$ is substantially inactive to human body and hence it does not exhibit the aforementioned desired pharmaceutical effects.

Japanese patent application publication No. 20,396/72 describes a method for producing coenzyme Q by cultivating a yeast, genus Candida capable of producing coenzyme Q in a culture medium containing n-alkane and POBA or POBA and acetic acid or its salt. However, production of coenzyme $Q_{10}$ is not disclosed and production of $Q_9$ or $Q_7$ as disclosed is limited due to the feature of using a yeast, genus Candida and to the fact that POBA has generally such a strong toxic property e.g. bactericidal property to microorganisms that it can be used as a food-preservative so that its addition is restricted to 5 mg/l at the most. Therefore, the method has not been a practical industrial method of producing coenzyme $Q_{10}$.

Japanese patent application publication No. 19,034/76 describes a method for producing coenzyme $Q_{10}$ by cultivating a microorganism of genus Alcaligenes, Trichosporon or Aureobasidium in a nutrient culture medium. However, the method has deficiencies of necessitating a pretreatment step of steam sterilizing the culture medium and thus necessitating a complicated production process.

An object of the present invention is to provide a method for producing coenzyme $Q_{10}$ in high yield in a culture medium containing a large quantity of POBA.

Another object of the present invention is to provide a method for producing coenzyme $Q_{10}$ quickly in a culture medium containing a large quantity of POBA.

Still another object of the present invention is to provide a method for producing coenzyme $Q_{10}$ in a simple industrial process in a culture medium containing a large quantity of POBA.

The present invention provides a method for producing coenzyme $Q_{10}$, comprising cultivating a microorganism selected from the strains Aureobasidium sp. No. 14 (FERM BP—1) and Trichosporon sp. WY 2—2 (FERM BP—2) in a culture medium containing p-hydroxybenzoic acid at a concentration of 1,000—4,000 $\mu$g/ml and 250—1,000 $\mu$g/ml, respectively, and at a temperature of 20—37°C, and recovering the coenzyme $Q_{10}$ thus produced.

According to the method of the present invention, the culture medium can also contain ethanol as a main carbon source in addition to POBA.

In the description which follows, reference will be made to the accompanying drawings in which:

Figures 1 to 5 are characteristic graphs showing the relationship between concentrations of POBA and production of coenzyme $Q_{10}$, corresponding respectively to data in Examples 1 to 5 which will be described later in detail.

We have made various experiments and studies on relations between nutrient culture media and microorganisms for producing coenzyme $Q_{10}$, particularly of genera Aureobasidium and Trichosporon, leading to a finding that the microorganisms Aureobasidium sp. No. 14 and Trichosporon WY 2—2 do not die even when POBA is added in such an amount that exceeds far beyond generally used. To the contrary, the productivity of coenzyme $Q_{10}$ is increased. It is quite surprising that microorganisms Aureobasidium sp. No. 14 and Trichosporon WY 2—2 can tolerate a high concentration of POBA, grow and even produce coenzyme $Q_{10}$ in exceedingly high yield.

Aureobasidium sp. No. 14 strain has been deposited at the Fermentation Research Institute (Fermentation Research Institute, Agency of Industrial Science and Technology, Ministry of International Trade and Industry) under deposit number FERM BP—1. We reported orally at the Annual Meeting of the Society of Japanese Agricultural Chemistry held in 1977 that the Aureobasidium sp. No. 14 strain exhibits strong tolerance to phenols and can utilize POBA as a sole carbon source, and filed a patent application which was published as Japanese patent application publication No. 54—45963. However, merely the assimilation or oxidization of phenols and growth of the Aureobasidium sp. No. 14 strain was reported at the 1977 meeting. There was no mention of the production of coenzyme $Q_{10}$.

We have deposited Trichosporon sp. WY 2—2 strain at the Fermentation Research Institute under deposit number FERM BP—2.

Aureobasidium Sp. No. 14, also known as Aureobasidium pullulans, has the following characteristics:

I. Culture characteristics

(1) Malt extract agar:

(a) *Slant culture* Growth was abundant after 2 days at 30°C and faster than on the potato dextrose agar slant. Olive green, echinulate, beaded or spreading colonies without luster grew along inoculated streak. They were a little raised, flat and yeasty mass mixed with aerial hyphae; such fungal growth were more abundant at the lower part of the slant. After 3 days the whole of the lower part of the slant was covered with olive green fungal mycelia. Incubation at 30°C for 5 days gave rise to a more abundant growth of olive green hyphal mat, covering the slant surface from the bottom to the middle of the slant. At the top of the slant, floccose hyphae were elongated, being intertangled with black yeasty colonies along the inoculated streak. The reverse of the slant became greenish-black.

(b) *Malt extract agar: Giant colony* Characteristics of these colonies were almost the same as those on potato dextrose agar: irregularly circular and of lobate or filamentous margin. The marginal black yeast portions were a little lustrous caused by the coexistence of scanty hyphae.

(2) Malt extract-peptone-yeast extract agar:

(a) *Slant culture* Shiny colonies similar to those of malt extract agar slant were formed within at least 2 days. However, these consisted solely of slimy, creamy and yeast like mass and no fungal mycelium could be seen at all; the reverse was deep black.

(3) Potato dextrose agar:

(a) *Slant culture* Growth was somewhat retarded in this medium as compared to the two other media. Scanty filiform or echinulate colonies were seen along inoculated streak after 2 days at 30°C. Colonies were composed of yeasty mat, at first greenish-white, later becoming greenish-black, or black; flat and a little raised, the margin being irregular. The mat was a little lustrous, mucoid and creamy. Occasionally very tiny, grey or olive green hyphal mycelia appeared especially underneath the slant. After 3 days the fungal growth increased becoming dark olive green to dark green. After 5 days the whole of the lower part of the slant was covered with floccose aerial hyphae; however the upper surface still had a black-yeasty colony, mainly along the inoculated streak. The reverse of the slant was greenish-black.

(b) *Potato dextrose agar: Giant colony* Colony grew slower than that on the agar slant, attained a diameter of about 1.5—1.7 cm after 7 days at 30°C, and appeared raised or umbonate form. It was composed of a soft, slimy, black yeasty portion and a dark greenish-grey fungal portion. The latter was mainly floccose radiated from the center of the colony; the former was developed at the marginal part of the colony and was also sparsely scattered throughout the fungal part.

II. Physiological properties

(1) Optimum culturing condition

| | |
|---|---|
| optimum pH for growth | 5.4—6.3 |
| optimum culturing temperature | 30—35°C |

(2) range for culturing

| | |
|---|---|
| range of pH for culturing | 2.9—8.9 |
| range of temperature for culturing | 10—45°C |

(3) Microscopic morphology

By slide culture and the usual microscopic preparations of the organism grown on malt extract agar or potato dextrose agar, the following morphological characteristics were observed.

*Yeast-like cells.* Under the microscope cells in the yeasty colonies on agar culture were observed as ellipsoidal, fusoid, ovate or spherical single separate cells. Often chains or masses thereof by budding were also seen. Cells were light olive to green and hyaline, 1.5—4 × 4—10 μm in size.

*Hyphae.* From ground mass formed by such yeast-like cells, slender filamentous hyphae began to elongate as cultivation proceeded. They were hyaline and light olive to olive green, and had transverse septa without clamp connection. One cell in the hyphae was 1.5—2.5 × 20 μm or more in size. Young hyphae were thin-walled but maturing areas became dark-brown and thick-walled. Hyphal cells sometimes bore arthroconidial structure at the distal regions.

*Conidia.* Many undifferentiated condiogenous cells were produced on hyphae laterally, terminally or intercalarily. Blastic condia were formed simultaneously in dense groups. They were hyaline, light olive to olive green, smooth, one-celled rather variable in shape and size, but neither coiled nor branched. When sporulation was abundant they were produced all over the cell surface. Conidia arose directly as buds from vegetative hyphae, and no conidiophores, phialides or pegs could be seen. Often acropetalous conidial chains were present as a result of budding. Finally, conidia detached from hyphae, and proliferated as separate yeast cells.

Identification

Considering the foregoing experimental results, the mycological identification of strain No. 14 was determined as Aureobasidium pullulans by the following references of

(1) Commonwealth Mycological Institute (edited by G.C. Ainsworth); Dictionary of the Fungi, Vol. 6, 1971.
(2) Illustrated Genera of Imperfect Fungi, Vol. 3, 1972; edited by H.L. Barnett and B.B. Hunter.
(3) Mycopathologia, Vol. 12, page 1, 1959; reported by W.M.B. Cooke.

Taxonomical studies on Trichosporon WY 2—2 are described in J. Ferment. Technol., vol 47, no. 3, p 161—166 (1969). The characteristics of Trichosporon Sp. WY 2—2 are as follows.

I. Morphological and culture characteristics of WY 2—2.

| | | |
|---|---|---|
| Growth in malt extract | | |
| | After 3 days at 28°C | Round to oval cells (1.0—4.0) ×(2.0—14.0) μ, cylindrical (1.5—4.0) × (5.0—24.0) μ, a pellicle, a sediment. |
| | After one month at 20°C | Very thick pellicle and a sediment. |
| Streak culture on malt agar | | White to cream-colored, raised, wrinkled, partly hairy, wet with dull and shiny parts. |
| Slide culture, potato agar | | Good development mycelium. The mycelium is split up into arthrospores of various length. |
| Sporulation | | Not observed. |

II. Physiological properties of WY 2—2 and T. cutaneum.

| | WY 2—2 | T. cutaneum |
|---|---|---|
| Fermentation | absent | absent |
| Sugar assimilation | | |
| glucose | + | + |
| maltose | + | + |
| galactose | + | + |
| lactose | + | + |
| saccharose | + | + |
| fructose | + | + |
| mannose | + | + |
| xylose | + | NT |
| arabinose | + | NT |
| dextrin | + | NT |
| α-Methyl D-glucoside | + | NT |
| Assimilation of $KNO_3$ | negative | negative |

| | WY 2—2 | T. cutaneum |
|---|---|---|
| Utilization of ethanol as sole source of carbon | growth, but required Fe ion | growth |
| Splitting arbutin | weakly positive | weakly positive |
| Production of carotenoid pigments | negative | negative |
| Production of starch-like compounds | negative | negative |
| Action in BCP milk | unchanged, weakly acudic | unchanged |
| Fat splitting | positive | negative |
| Growth in vitamin-free medium | negative | negative |
| Vitamins stimulating growth | thiamine and biotin | NT |
| pH range for growth | growth at 3.5—10.0 | growth at 3.5—10.0 |
| Optimum temperature | 25°—30° | 25°—30° |
| Relation to free oxygen | aerobic | aerobic |
| Catalase | positive | positive |
| Source | isolated from microbially treated sulphate sludge of petroleum | IFO 0116 |

NT: Not tested.
IFO: Institute for Fermentation, Osaka.

In the present invention POBA is added in a concentration of about 1,000—4,000 μg/ml to a culture medium at a temperature of about 20—37°C when the microorganism Auerobasidium sp. No. 14 is used. If the concentration is less than about 1,000 μg/ml, the effect of addition can not be exhibited sufficiently to increase the production of coenzyme $Q_{10}$, while if the concentration is larger than about 4,000 μg/ml, the bactericidal property of POBA is exhibited so much and the growth is suppressed so that the growth rate decreases. A concentration of about 1,500—3,500 μg/ml is particularly preferable. As for the microorganism Trichosporon WY 2—2, we have found out that POBA is added in a concentration of about 250—1,000 μg/ml to culture medium at a temperature of about 20—37°C. If the concentration is less than about 250 μg/ml, the production of coenzyme $Q_{10}$ decreases, while if the concentration is larger than about 1,000 μg/ml, the bactericidal property of POBA is exhibited so much and the growth rate decreases. A concentration of about 400—800 μg/ml is particularly preferable.

According to the present invention, any carbon source can be used as far as the microorganisms concerning the present invention can assimilate. For instance, use is made of carbohydrates such as starch etc.; alcohols such as glycerol, propanol etc.; sugars such as glucose, sucrose, molasses etc.; hydrocarbons such as aliphatic and aromatic hydrocarbons; or organic acids such as palmitic acid, fumalic acid etc.

We have found out also that coenzyme $Q_{10}$ can be produced not only in extremely short period of time but also in exceedingly high yield, if ethanol is used as a carbon source in addition to POBA.

Accordingly, in another aspect of the present invention, the method of the present invention produces coenzyme $Q_{10}$ by effecting cultivation in a culture medium containing ethanol as a main carbon source in addition to the large quantity of POBA.

It is quite surprising that, if ethanol is added to the culture medium, the growth rate increases remarkably to about twice as much of usual method so that coenzyme $Q_{10}$ can be produced extremely quickly despite the bactericidal property of ethanol.

The concentration of ethanol to be added is about 0.5—4.0% (V/V) to the culture medium. If the concentration is less than about 0.5% (V/V), the amount of the cells produced becomes less. If the concentration is larger than about 4.0% (V/V), the bactericidal property of ethanol is exhibited so much and the growth is suppressed to decrease the growth rate. A concentration of about 1.0—3.0% (V/V) is particularly preferable.

In the present invention, the usual nitrogen source, vitamines, inorganic salts etc. generally used can be added in usual amounts to the culture medium.

Preferable cultivation conditions are a pH of about 2—8, a temperature of about 20—37°C, the cultivation period of about 20—80 hrs. and an aerobic atmosphere.

If pH value varies noticeably during cultivation to suppress the growth of the cells, pH is preferably adjusted by addition of acidic or alkaline material.

The following Examples illustrate the present invention.

In the succeeding Examples, all quantities of materials are shown by gram unit, unless otherwise specified.

Example 1

In this Example, Aureobasidium sp. No. 14 strain is used for producing coenzyme $Q_{10}$.

Inocolum cells are cultivated by the MPY medium which has the following composition.

Composition of the MPY medium:

| | |
|---|---|
| Malt extract broth | 30 |
| Peptone | 5 |
| Yeast extract | 0.1 |
| Tap water | 1 l |

The culture medium used for pre-culture and main culture has the following composition.

Composition of culture medium used for pre-culture and main culture:

| | |
|---|---|
| $NH_4NO_3$ | 5.0 |
| $KH_2PO_4$ | 2.5 |
| $MgSO_4 \cdot 7H_2O$ | 1.0 |
| NaCl | 0.1 |
| Yeast extract | 0.1 |
| $CaCl_2 \cdot 2H_2O$ | 0.01 |
| $FeCl_3 \cdot 6H_2O$ | 0.01 |
| $C_2H_5OH$ | 20 ml |
| POBA | 0 (pre-culture)<br>0 — 2,000 $\mu$g/ml<br>(main culture) |
| Tap water | 980 ml |
| pH | 5.0 |

Cultivations are effected as follows.

A loopful cells from the young slant culture is inoculated to the MPY medium 10 ml, and cultivated at 30°C for 7 days to obtain inoculum cells.

A drop of the inoculum cell suspension is inoculated to 200 ml of the above-mentioned pre-culture medium not containing POBA and subsequently subjected to shaking cultivation at 30°C for 48 hrs. to yield a pre-culture broth.

The pre-culture broth is added to 800 ml of the aforementioned main culture medium containing POBA in a concentration of 0—2,000 μg/ml in a volume ratio of 1:4 to a total volume of 1 l and subjected again to shaking cultivation at 30°C for 48 hrs.

The grown cells are separated from supernatant solution by centrifugal precipitation of the culture broth and washed three times with distilled water. Suspension of wet cells thus obtained are treated in the following procedure to give a raw sample for extracting coenzyme $Q_{10}$.

The mixture of 50 ml of the suspension of the wet cells, 150 ml of methanol, 5 g of pyrogallol and 20 g of sodium hydroxide are subjected to saponification by treating under a reflux condenser at 90°C for 1 hr. and then extracted twice with each 100 ml of n-hexane. The n-hexane layer of total 200 ml is washed three times with distilled water, then dehydrated and dried overnight with anhydrous sodium sulfate, and thereafter concentrated under reduced pressure. The sample thus obtained is used for the quantitative analysis of coenzyme $Q_{10}$ in the following way.

The concentrate is dissolved in 7 ml of ethanol to give a sample solution.

2 ml of the sample solution, 0.5 ml of ethylcyanoacetate (ECA) and 0.5 ml of 0.2N-KOH aqueous solution are mixed. After exactly 10 min., Optional Density at 625 nm ($OD_{625}$) is determined. As a control, $OD_{625}$ is determined for a similar solution wherein instead of ECA an equal amount of ethanol is used and reacted in the same way. The control value is subtracted from the above determined value. The results are shown in Table 1.

TABLE 1

(Analytical results)

| POBA concentration (μg/ml) | Amount* of produced coenzyme $Q_{10}$ (μg) | Dry cell weight (g) | $Q_{10}$ production (μg) |
|---|---|---|---|
| | 1 l culture broth | 1 l culture broth | 1 g dry cell weight |
| 0 | 770 | 3.5 | 220 |
| 100 | 980 | 3.6 | 270 |
| 500 | 980 | 3.6 | 270 |
| 1,000 | 1,190 | 3.7 | 320 |
| 2,000 | 1,820 | 2.9 | 630 |

* : Amount of coenzyme $Q_{10}$ obtained from 1 l culture broth.

The above results are also shown as characteristic graphs in the attached Figs. 1A and 1B.

Example 2

In this Example, the same microorganism and cultivation conditions as in Example 1 are used, and the same treatments as in Example 1 are effected, except that main culture is not adjusted to the pH value of 5.0 and conducted in an acidic condition of a pH value of 2.5—4.

A loopful cells from the young slant culture is inoculated to the MPY medium 10 ml, and cultivated at 30°C for 7 days to obtain onoculum cells.

A drop of the inoculum cell suspension is inoculated to 200 ml of the pre-culture medium not containing POBA and subsequently subjected to shaking cultivation at 30°C for 48 hrs. to yield a pre-culture broth.

The pre-culture broth is added to 800 ml of the main culture medium containing POBA in a concentration of 0—2,000 μg/ml and a pH value of 2.5—4.0 depending on POBA concentrations in a volume ratio of 1:4 to a total volume of 1 l and subjected again to shaking cultivation at 30°C for 48 hrs.

The cells are separated from supernatant solution by centrifugal precipitation of the culture broth and washed three times with distilled water. Suspension of wet cells thus obtained are treated in the following procedure to give a raw sample for extracting coenzyme $Q_{10}$.

The mixture of 50 ml of the suspension of the wet cells, 150 ml of methanol, 5 g of pyrogallol and 20 g of sodium hydroxide are subjected to saponification by treating under a reflux condenser at 90°C for 1 hr. and then extracted twice with each 100 ml of n-hexane. The n-hexane layer of total 200 ml is washed three times with distilled water, then dehydrated and dried overnight with anhydrous sodium sulfate, and thereafter concentrated under reduced pressure. The sample thus obtained is used for quantitative analysis of coenzyme $Q_{10}$ in the same way as in Example 1. The results are shown in Table 2.

TABLE 2

(Analytical results)

| POBA concentration ($\mu$g/ml) | Amount* of produced coenzyme $Q_{10}$ ($\mu$g) | Dry cell weight (g) | $Q_{10}$ production ($\mu$g) |
|---|---|---|---|
| | 1 l culture broth | 1 l culture broth | 1 g dry cell weight |
| 0 | 700 | 2.5 | 280 |
| 100 | 840 | 2.6 | 320 |
| 500 | 1,120 | 2.1 | 530 |
| 1,000 | 1,190 | 2.1 | 570 |
| 2,000 | not grown | — | — |

*: Amount of coenzyme $Q_{10}$ obtained from 1 l culture broth.

The above results are also shown as characteristic graphs in the attached Figs. 2A and 2B.

Example 3

The same experiment as in Example 1 is repeated, except that a loopful cells from the young slant culture is inoculated to 10 ml of the preculture medium not containing POBA instead of the MPY medium and cultivated at 30°C for 10 days to obtain inoculum cells, and that POBA concentrations in the main culture medium is raised up.

A drop of the inoculum cell suspension is inoculated to 200 ml of the preculture medium not containing POBA and subsequently subjected to shaking cultivation at 30°C for 48 hrs. to yield a pre-culture broth.

The pre-culture broth is added to 800 ml of the main culture medium containing POBA in a concentration of 1,000—4,000 $\mu$g/ml in a volume ratio of 1:4 to a total volume of 1 l and subjected again to shaking cultivation at 30°C for 48 hrs.

The grown cells are separated from supernatant solution by centrifugal precipitation of the culture broth and washed three times with distilled water. Suspension of wet cells thus obtained are treated in the following procedure to give a raw sample for extracting coenzyme $Q_{10}$.

The mixture of 50 ml of the suspension of the wet cells, 150 ml of methanol, 5 g of pyragallol and 20 g of sodium hydroxide are subjected to saponification by treating under a reflux condenser at 90°C for 1 hr. and then extracted twice with each 100 ml of n-hexane. The n-hexane layer of total 200 ml is washed three times with distilled water, then dehydrated and dried overnight with anhydrous sodium sulfate, and thereafter concentrated under reduced pressure. The sample thus obtained is used for quantitative analysis of coenzyme $Q_{10}$ in the same way as in Example 1.

The results are shown in Table 3.

TABLE 3

(Analytical results)

| POBA concentration ($\mu g/ml$) | Amount* of produced coenzyme $Q_{10}$ ($\mu g$) | Dry cell weight (g) | $Q_{10}$ production ($\mu g$) |
|---|---|---|---|
| | 1 l culture broth | 1 l culture broth | 1 g dry cell weight |
| 1,000 | 2,310 | 7.1 | 330 |
| 2,000 | 3,920 | 5.5 | 710 |
| 3,300 | 3,220 | 5.8 | 560 |
| 4,000 | 2,940 | 7.3 | 400 |

*: Amount of coenzyme $Q_{10}$ obtained from 1 l culture broth.

The above results are also shown as characteristic graphs in the attached Figs. 3A and 3B.

Example 4

The same experiment as in Example 3 is repeated, except that a loopful cells from the young slant culture is cultivated for 7 days instead of 10 days and that a temperature of 37°C is used for the main cultivation instead of 30°C.

The results are shown in Table 4.

TABLE 4

(Analytical results)

| POBA concentration ($\mu g/ml$) | Amount* of produced coenzyme $Q_{10}$ ($\mu g$) | Dry cell weight (g) | $Q_{10}$ production ($\mu g$) |
|---|---|---|---|
| | 1 l culture broth | 1 l culture broth | 1 g dry cell weight |
| 1,000 | 1,120 | 8.4 | 130 |
| 2,000 | 2,520 | 10.4 | 240 |
| 2,700 | 3,220 | 9.2 | 350 |
| 3,300 | 3,080 | 7.2 | 430 |
| 4,000 | 280 | 2.4 | 120 |

*: Amount of coenzyme $Q_{10}$ obtained from 1 l culture broth.

The above results are also shown as characteristic graphs in the attached Figs. 4A and 4B.

Example 5

In this Example, coenzyme $Q_{10}$ is produced using Trichosporon sp. WY 2—2 strain instead of Aureobasidium sp. No. 14 strain.

Cultivations are effected as follows.

A loopful cells from the young slant culture is inoculated to the aforementioned MPY medium 10 ml and cultivated at 30°C for 7 days to obtain inoculum cells.

A drop of the inoculum cell suspension is inoculated to 200 ml of the above-mentioned pre-culture medium not containing POBA and subsequently subjected to shaking cultivation at 30°C for 48 hrs. to yield a pre-culture broth.

The pre-culture broth is added to 800 ml of the aforementioned main culture medium containing POBA in a concentration of 0—2,000 μg/ml in a volume ratio of 1:4 to a total volume of 1 l and subjected again to shaking cultivation at 30°C for 48 hrs.

The cells are separated from supernatant solution by centrifugal precipitation of the culture broth and washed three times with distilled water. Suspension of wet cells thus obtained is treated in the following procedure to give a raw sample for extracting coenzyme $Q_{10}$.

The mixture of 50 ml of the suspension of the wet cells, 150 ml of methanol, 5 g of pyrogallol and 20 g of sodium hydroxide are subjected to saponification by treating under a reflux condenser at 90°C for 1 hr. and then extracted twice with each 100 ml of n-hexane. The n-hexane layer of total of 200 ml is washed three times with distilled water, then dehydrated and dried overnight with anhydrous sodium sulfate, and thereafter concentrated under reduced pressure. The sample thus obtained is used for quantitative analysis of coenzyme $Q_{10}$ in the following way.

The concentrate is dissolved in 2 ml of acetone to give a sample solution. Ten μl of the sample solution is used for the quantitative analysis by high performance liquid chromatography. The quantitative value is determined by the standard curve obtained from the authentic specimen.

The results are shown in Table 5.

TABLE 5

(Analytical results)

| POBA concentration (μg/ml) | Amount* of produced coenzyme $Q_{10}$ (μg) | Dry cell weight (g) | $Q_{10}$ production (μg) |
|---|---|---|---|
| | 1 l culture broth | 1 l culture broth | 1 g dry cell weight |
| 0 | 6.03 | 0.053 | 114.0 |
| 100 | 9.50 | 0.098 | 97.0 |
| 500 | 17.80 | 0.173 | 103.0 |
| 1,000 | 13.20 | 0.190 | 69.4 |
| 2,000 | 8.52 | 0.181 | 47.0 |

* : Amount of coenzyme $Q_{10}$ obtained from 1 l culture broth.

The above results are also shown as characteristic graphs in the attached Figs. 5A and 5B.

Referential Example

Growth characteristics of Auerobasidium sp. No. 14 strain is studied on various utilizable main carbon sources other than ethanol. The main carbon sources used are glycerol, glucose, n-paraffin mixture (Carbon number $C_{11}$~$C_{14}$), n-propanol, acetone, ethyl acetate, cyclohexanol, n-dodecane or palmitic acid.

Cultivations are effected as follows.

A drop of the inoculum cell suspension obtained in the same procedure as in Example 1 is inoculated to 10 ml of a culture medium which has the same composition employed in Example 1 for pre-culture, except that as a main carbon source 20 ml of ethanol is replaced by 20 g or 20 ml of the above mentioned materials.

When the main carbon source used is insoluble in water, 0.5 g surfactant, Tween 80 is added per 1 l medium.

The inoculated culture medium is subjected to shaking cultivation at 30°C and cell growth expressed in $OD_{660}$ unit and the cultivation time are observed.

The results are shown in Table 6.

TABLE 6

(Growth characteristics on various main carbon sources)

| Main carbon source | Growth ($OD_{660}$) | Cultivation time (hrs.) |
|---|---|---|
| glycerol | 4.14 | 54 |
| glucose | 4.46 | 54 |
| n-paraffin ($C_{11}\sim C_{14}$) | 2.86 | 54 |
| n-propanol | 2.50 | 248 |
| acetone | 1.02 | 248 |
| ethylacetate | 2.29 | 96 |
| cyclohexanol | 0.17 | 224 |
| n-dodecane | 3.54 | 54 |
| palmitic acid | 3.64 | 73 |

Example 6

In this Example, coenzyme $Q_{10}$ is produced by Aureobasidium sp. No. 14 strain grown in the media containing various main carbon sources and a large quantity of POBA, 2,000 $\mu$g/ml. The main carbon sources used are glycerol, glucose or n-paraffin ($C_1\sim C_{14}$).

Cultivation is effected in the same way as in Referential Example to obtain 100 ml of a pre-culture broth.

The pre-culture broth thus obtained is added to 400 ml of the culture medium described in Referential Example containing POBA in the concentration of 2,000 $\mu$g/ml in a volume ratio of 1:4 to a total volume of 500 ml and subjected again to shaking cultivation at 30°C for a given period.

The grown cells are separated from supernatant solution by centrifugal precipitation of the culture broth and washed three times with distilled water. Suspension of wet cells thus obtained are treated in the same procedure as in Example 1 to give a raw sample for extracting and quantitative analysis of coenzyme $Q_{10}$.

The results are shown in Table 7.

TABLE 7

(Production of $Q_{10}$ on various main carbon sources)

| Main carbon source | Pre-culture (hrs.) | Main culture (hrs.) | Wet cell weight (g) | $Q_{10}$ ($\mu$g/l) |
|---|---|---|---|---|
| glycerol | 42 | 52 | 14.2 | 2,900 |
| glucose | 47 | 47 | 10.0 | 1,900 |
| n-paraffin ($C_{11}\sim C_{14}$) | 47 | 70 | 1.4 | 100 |

As clearly indicated from the above quantitative analytical results, the present invention is exceedingly superior to conventional methods.

**Claims**

1. A method for producing coenzyme $Q_{10}$, comprising cultivating a microorganism selected from the strains Aureobasidium sp. No. 14 (FERM BP—1) and Trichosporon sp. WY 2—2 (FERM BP—2) in a culture medium containing p-hydroxybenzoic acid at a concentration of 1,000—4,000 $\mu$g/ml and 250—1,000 $\mu$g/ml, respectively, and at a temperature of 20—37°C, and recovering the coenzyme $Q_{10}$ thus produced.

2. A method according to claim 1, wherein strain Aureobasidium sp. No. 14 (FERM BP—1) is cultivated in a culture medium containing p-hydroxybenzoic acid at a concentration of 1,500—3,500 $\mu$g/ml.

3. A method according to claim 1, wherein the strain Trichosporon WY 2—2 (FERM BP—2) is cultivated in a culture medium containing p-hydroxybenzoic acid at a concentration of 400—800 $\mu$g/ml.

4. A method according to any one of the preceding claims, wherein cultivation is effected at a pH value of 2—8.

5. A method according to any one of the preceding claims, wherein cultivation is effected in an aerobic atmosphere for 20—80 hrs.

6. A method according to any one of the preceding claims, wherein an acidic material or alkaline material is added to the culture medium during cultivation.

7. A method according to any one of the preceding claims, wherein the culture medium contains ethanol as the main carbon source.

8. A method according to claim 7, wherein ethanol is added to the culture medium in a concentration of 0.5—4.0% by volume.

9. A method according to claim 8, wherein ethanol is added to the culture medium in a concentration of 1.0—3.0% by volume.

**Revendications**

1. Procédé de préparation de coenzyme Q 10, qui consiste à cultiver un microorganisme choisi dans les souches Aureobasidium sp. No14 (FERM BP—1) et Trichosporon sp. WY 2—2 (FERM BP—2) sur un milieu de culture contenant de l'acide p-hydroxybenzoïque à une concentration de 1000 à 4000 $\mu$g/ml et 250 à 1000 $\mu$g/ml respectivement, et à une température de 20 à 37°C, et à isoler le coenzyme Q 10 ainsi formé.

2. Procédé selon la revendication 1, dans lequel la souche Aureobasidium sp. No 14 (FERM BP—1) est cultivée sur un milieu contenant de l'acide p-hydroxybenzoïque à une concentration de 1500 à 3500 $\mu$g/ml.

3. Procédé selon la revendication 1, dans lequel la souche Trichosporon WY 2—2 (FERM BP—2) est cultivée sur un milieu de culture contenant de l'acide p-hydroxybenzoïque à une concentration de 400 à 800 $\mu$g/ml.

4. Procédé selon l'une quelconque des revendications précédentes, dans lequel la culture est effectuée à un pH de 2 à 8.

5. Procédé selon l'une quelconque des revendications précédentes, dans lequel la culture est effectuée en atmosphère aérobie pendant 20 à 80 heures.

6. Procédé selon l'une quelconque des revendications précédentes, dans lequel un produit acide ou un produit basique est ajouté au milieu de culture pendant la culture.

7. Procédé selon l'une quelconque des revendications précédentes, dans lequel le milieu de culture contient de l'éthanol comme principale source de carbone.

8. Procédé selon la revendication 7, dans lequel l'éthanol est ajouté au milieu de culture à une concentration comprise entre 0,5 et 4% en volume.

9. Procédé selon la revendication 8, dans lequel l'éthanol est ajouté au milieu de culture à une concentration comprise entre 1,0 et 3% en volume.

**Patentansprüche**

1. Verfahren zur Herstellung von Coenzym $Q_{10}$, dadurch gekennzeichnet, dass man einen Organismus, ausgewählt aus den Stämmen Aureobasidium sp. Nr. 14 (FERM BP—1) und Trichosporon sp. WY 2—2 (FERM BP—2) in einem Kulturmedium, enthaltend p-Hydroxybenzoesäure in einer Konzentration von 1.000 bis 4.000 $\mu$g/ml, bzw. 250 bis 1.000 $\mu$g/ml und be einer Temperatur von 20 bis 37°C kultiviert und das so gebildete Coenzym $Q_{10}$ gewinnt.

2. Verfahren gemäss Anspruch 1, dadurch gekennzeichnet, dass man den Stamm Aureobasidium sp. Nr. 14 (FERM BP—1) in einem Kulturmedium, welches p-Hydroxybenzoesäure in einer Konzentration von 1.500 bis 3.500 $\mu$g/ml enthält, kultiviert.

3. Verfahren gemäss Anspruch 1, dadurch gekennzeichnet, dass man den Stamm Trichosporon WY 2—2 (FERM BP—2) in einem Kulturmedium, welches p-Hydroxybenzoesäure in einer Konzentration von 400 bis 800 $\mu$g/ml enthält, kultiviert.

4. Verfahren gemäss einem der vorhergehenden Ansprüche, dadurch gekennzeichnet, dass man die Kultivierung bei einem pH-Wert von 2 bis 8 durchführt.

5. Verfahren gemäss einem der vorhergehenden Ansprüche, dadurch gekennzeichnet, dass man die Kultivierung in einer aeroben Atmosphäre während 20 bis 80 Stunden durchführt.

6. Verfahren gemäss einem der vorhergehenden Ansprüche, dadurch gekennzeichnet, dass man zu dem Kulturmedium während der Kultivierung ein saures oder alkalisches Material zugibt.

7. Verfahren gemäss einem der vorhergehenden Ansprüche, dadurch gekennzeichnet, dass das Kulturmedium Ethanol als Hauptkohlenstoffquelle enthält.

8. Verfahren gemäss Anspruch 7, dadurch gekennzeichnet, dass man Ethanol zu dem Kulturmedium in einer Konzentration von 0,5 bis 4,0 Vol.% zugibt.

9. Verfahren gemäss Anspruch 8, dadurch gekennzeichnet, dass man Ethanol dem Kulturmedium in einer Konzentration von 1,0 bis 3,0 Vol.% zugibt.

FIG. 1A

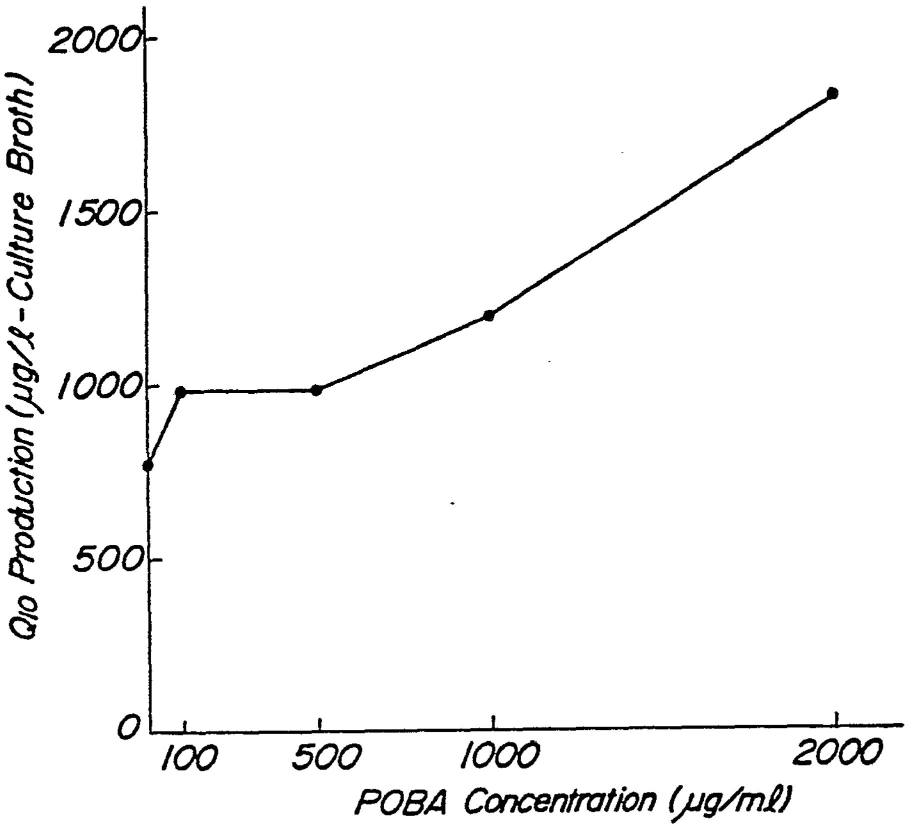
Q10 Production (μg/ℓ-Culture Broth)
2000
1500
1000
500
0
100
500
1000
2000
POBA Concentration (μg/mℓ)

FIG. 1B

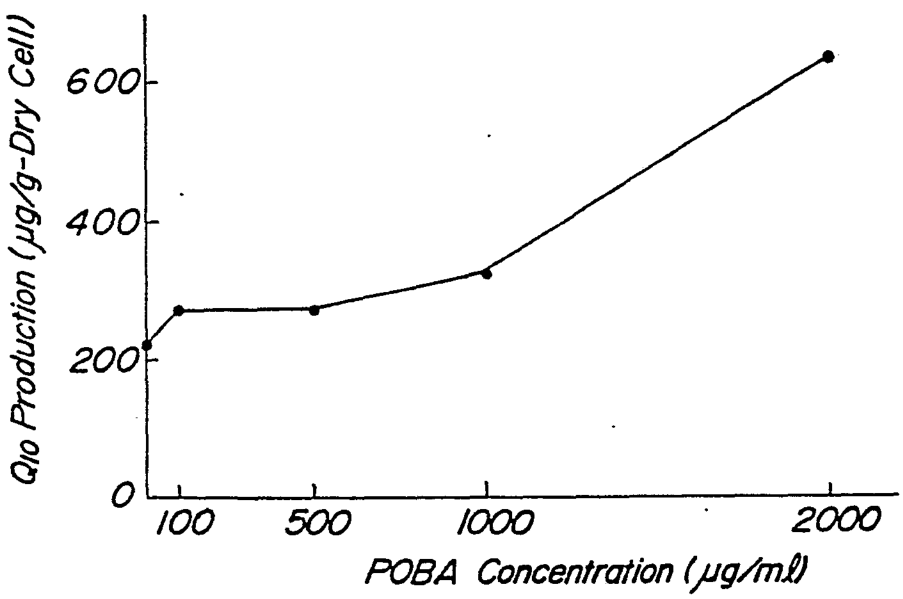
Q10 Production (μg/g-Dry Cell)
600
400
200
0
100
500
1000
2000
POBA Concentration (μg/mℓ)

FIG_2A

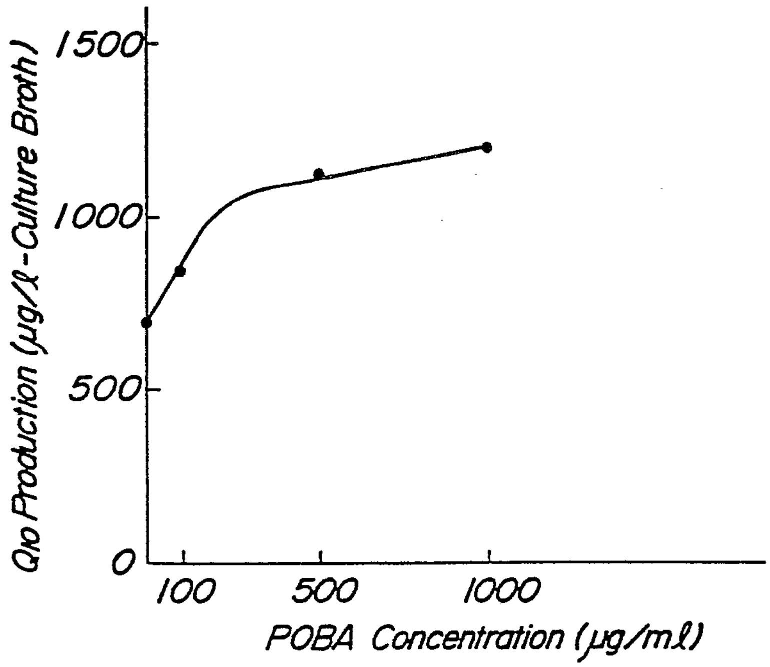
Q10 Production (μg/ℓ-Culture Broth)
1500
1000
500
0
100
500
1000
POBA Concentration (μg/mℓ)

FIG_2B

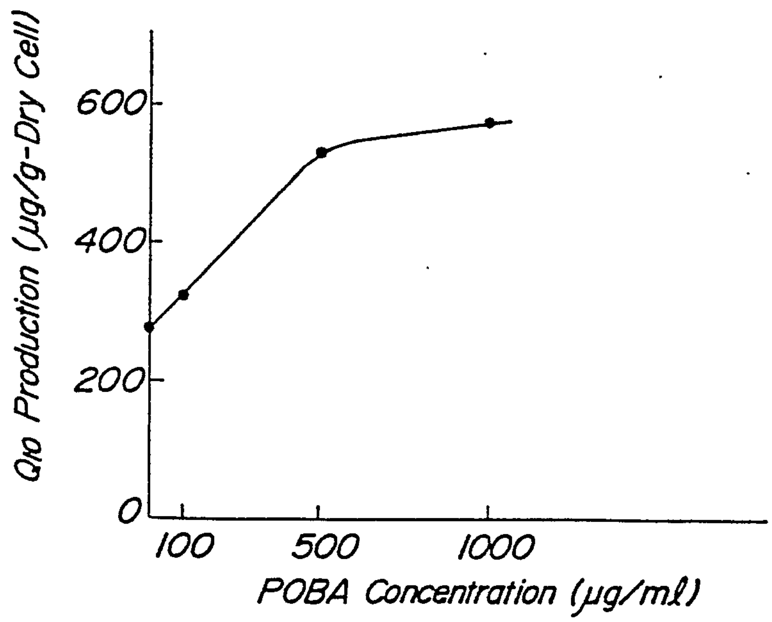
Q10 Production (μg/g-Dry Cell)
600
400
200
0
100
500
1000
POBA Concentration (μg/mℓ)

FIG. 3A

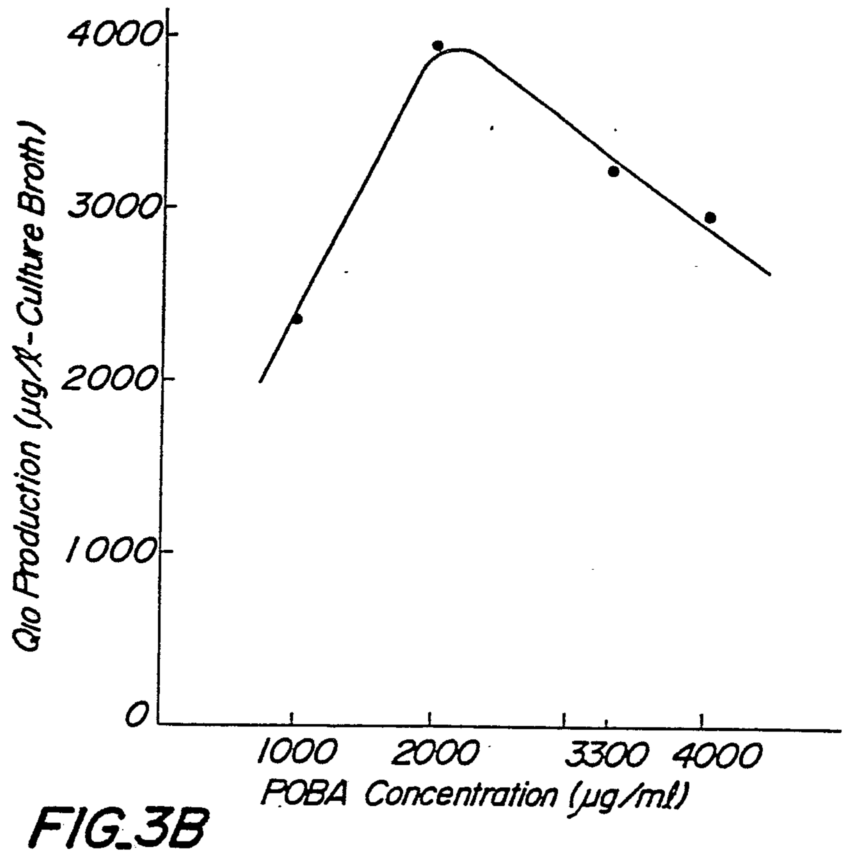
Q10 Production (µg/ℓ-Culture Broth)
4000
3000
2000
1000
0
1000
2000
3300
4000
POBA Concentration (µg/mℓ)

FIG. 3B

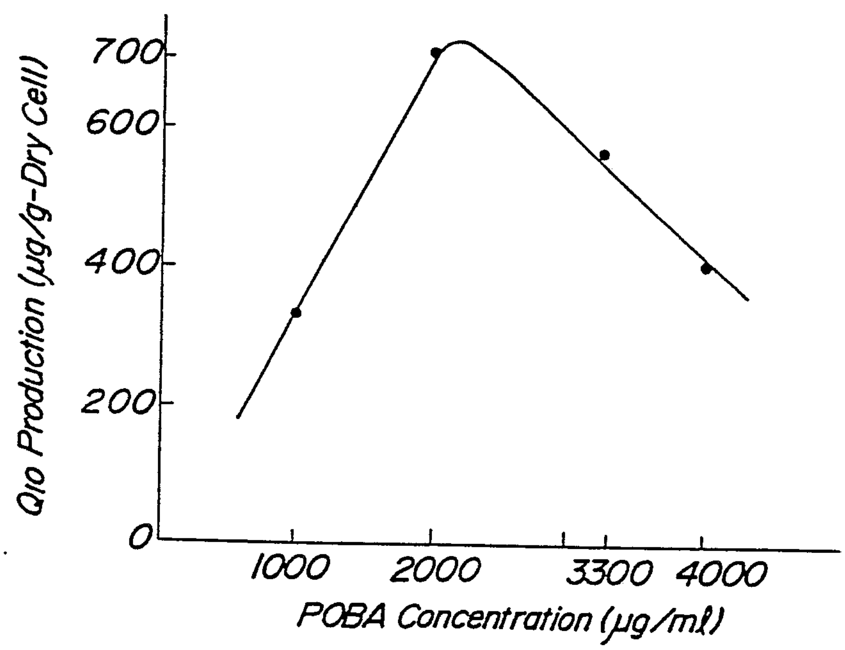
Q10 Production (µg/g-Dry Cell)
700
600
400
200
0
1000
2000
3300
4000
POBA Concentration (µg/mℓ)

FIG_4A

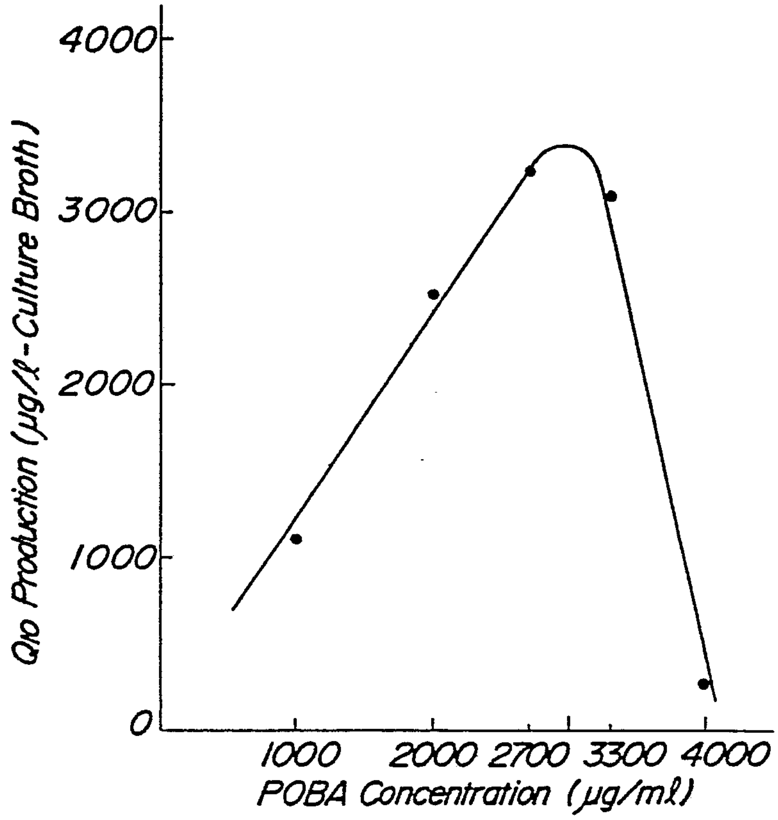
4000
3000
2000
1000
0
Q10 Production (μg/ℓ-Culture Broth)
1000
2000
2700
3300
4000
POBA Concentration (μg/mℓ)

FIG_4B

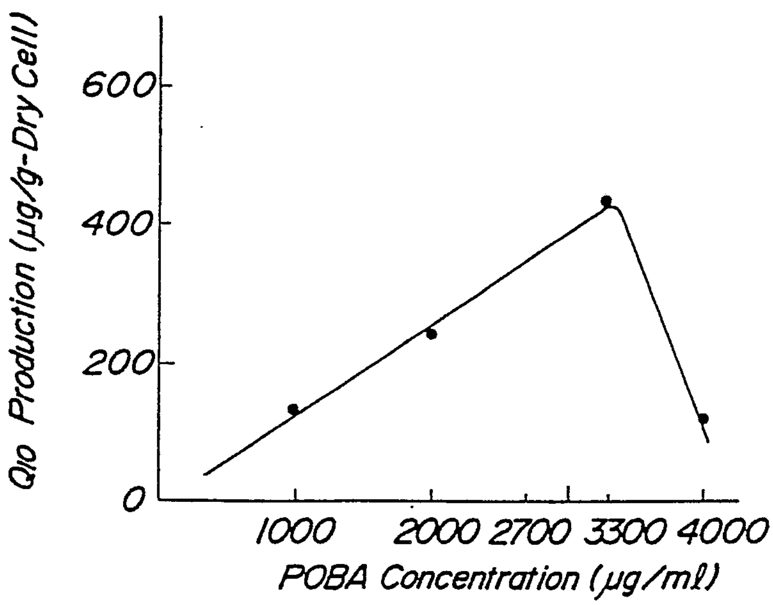
600
400
200
0
Q10 Production (μg/g-Dry Cell)
1000
2000
2700
3300
4000
POBA Concentration (μg/mℓ)

FIG_5A

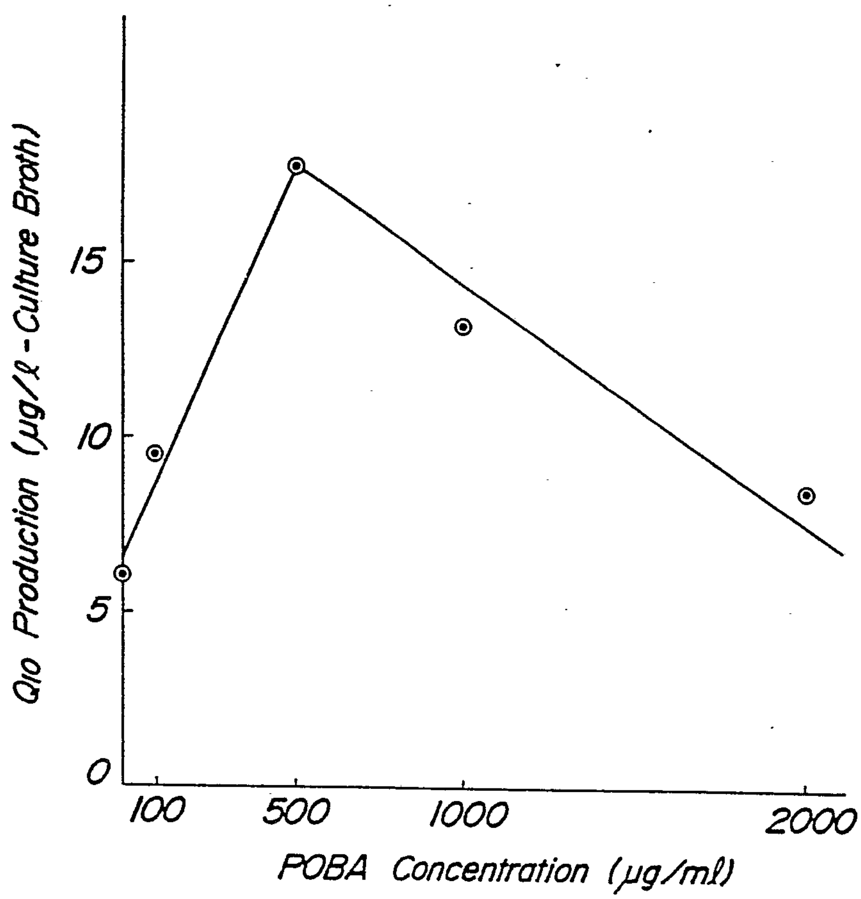
Q10 Production (µg/ℓ-Culture Broth)
15
10
5
0
100
500
1000
2000
POBA Concentration (µg/mℓ)

FIG.5B

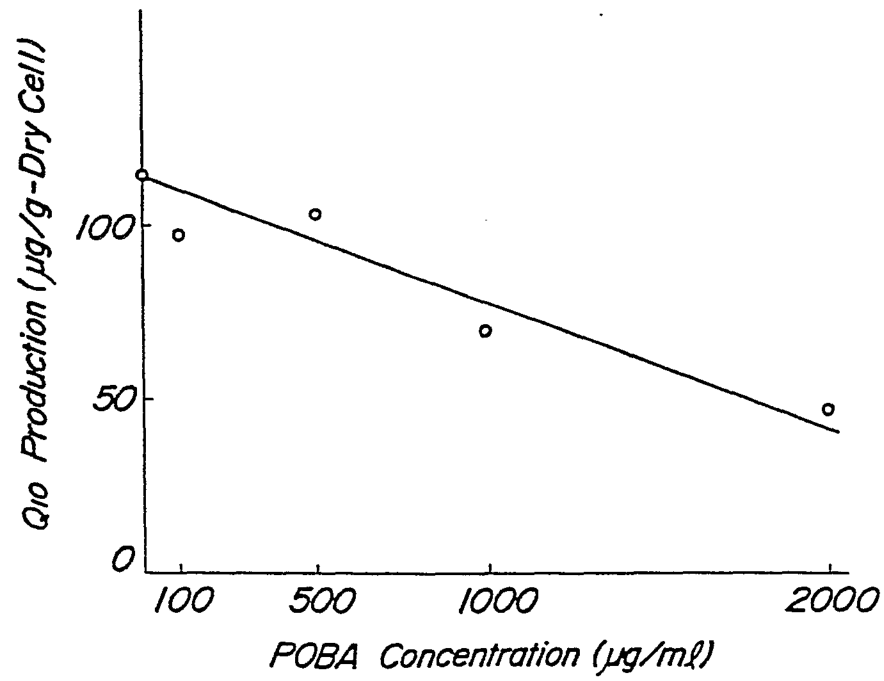
Q10 Production (μg/g-Dry Cell)
100
50
0
100
500
1000
2000
POBA Concentration (μg/mℓ)